# FASCICULE DE BREVET EUROPEEN

(11) **EP 0 730 573 B1**
(45) Date de publication et mention de la délivrance du brevet: **30.06.1999**
(21) Numéro de dépôt: 95932035.9
(22) Date de dépôt: 20.09.1995
(51) Int. Cl.: C07C 237/46, A61K 49/04

(54) **DERIVES IODES, LEUR PREPARATION ET LEUR APPLICATION COMME AGENTS DE CONTRASTE EN RADIOLOGIE X**
IODO-DERIVATE IHRE HERSTELLUNG UND VERWENDUNG ALS KONTRASTMITTEL FÜR RÖNTGEN-RADIOLOGIE
IODINATED DERIVATIVES, PREPARATION AND USE THEREOF AS CONTRAST AGENTS IN X-RAY RADIOLOGY

(30) Priorité: 22.09.1994 FR 9411326
(43) Date de publication de la demande: 11.09.1996
(73) Titulaire: GUERBET, 93420 Villepinte (FR)
(72) Inventeur: LE LEM, Gael, F-92210 Saint-Cloud (FR); MEYER, Dominique, F-94100 Saint-Maur (FR)
(74) Mandataire: Le Guen, Gérard
(86) Numéro de dépôt international: FR9501213
(87) Numéro de publication internationale: WO9609281

(56) Documents cités:
- EP-A- 0 354 836
- WO-A-92/18167
- WO-A-93/10824
- WO-A-94/21600
- WO-A-95/01966
- US-A- 4 065 553
- US-A- 4 065 554

## Description

La présente invention concerne des produits de contraste pour la radiologie par rayons X.

Ces molécules, de forte masse moléculaire puisqu'elles comportent au moins 12 noyaux phényles triiodés, ont une rémanence vasculaire nettement supérieure à celle des produits de contraste actuellement utilisés en clinique comme l'iobitridol ou l'iohexol- et plus ou moins analogue à celle des compositions résultant du greffage de noyaux iodés à des polymères, compositions dont on connait les inconvénients dus à la présence de molécules de masses moléculaires diverses dans un même échantillon. Ainsi on a constaté que cinq minutes après Injection intraveineuse chez le rat, d'une même dose en iode des produits de l'invention ou d'agents de contraste commercialisés, la concentration sanguine en iode des nouveaux composés était au moins trois fois plus élevée.

Par ailleurs, les solutions aqueuses de ces nouveaux composés ont une viscosité compatible avec leur administration intraveineuse chez l'homme, aux doses habituelles, tandis que leur osmolalité doit, en général, être relevée.

Les composés de l'invention sont représentés par la formule générale 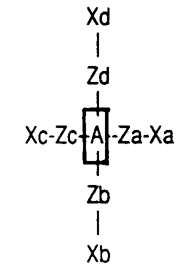 dans laquelle
Za, Zb, Zc, Zd, identiques ou différents, représentent Tz-Qz-Vz, Tz étant fixé sur A;
et Tz et Vz, identiques ou différents, représentent O, COND ou NDCO avec D représentant H, alkyle, hydroxyalkyle ou polyhydroxyalkyle en C₁ à C₆ et Qz représente alkylène, hydroxyalkylène ou polyhydroxyalkylène en C₁ à C₆, de préférence en C₁ à C₄, ou bien
Qz et/ou Tz n'existent pas;

- Xa, Xb, Xc Xd, identiques ou différents, représentent 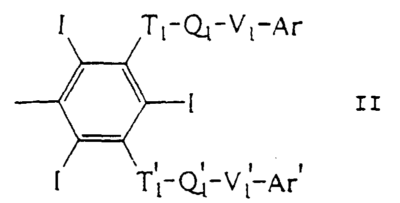 et
   · T₁, T'₁, V₁, V'₁,identiques ou différents, représentent CO-ND' ou ND'-CO avec D' représentant H ou alkyle en C₁ à C₆ portant, éventuellement, un ou plusieurs groupes OH;
   · Q₁ et Q'₁, identiques ou différents, représentent alkylène en C₁ à C₆, et de préférence en C₁ à C₄, portant événtuellement, un ou plusieurs groupes OH;
   · Ar et Ar', identiques ou différents, représentent:
- soit la formule III ou la formule IV 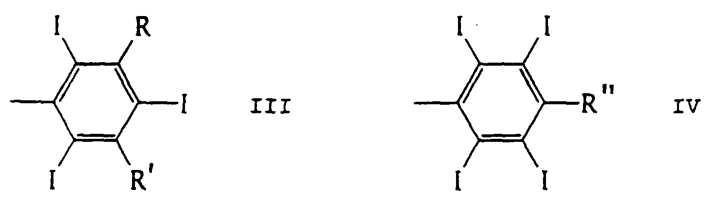 dans laquelle formule III, R est COOH et R' est CO-NR'₁R'₂ ou N(R'₁)-CO-R'₂, R'₁ et R'₂ étant H, alkyle, hydroxyalkyle ou polyhydroxyalkyle en C₁ à C₈ et comportant à eux deux plus de 4 OH; ou dans laquelle R et R', identiques ou différents, représentent CO-NR'₁R'₂ ou N(R'₁)-CO-R'₂ et R', et R'₂ sont H, alkyle, hydroxyalkyle ou polyhydroxyalkyle en C₁ à C₈ et R et R' comportent à eux deux plus de 6 groupes OH, ou mieux plus de 8 groupes OH et de préférence 10 groupes OH;
   et dans laquelle formule IV, R" représente CO-NR'₁R'₂ ou N(R'₁)-CO-R'₂, R'₁ et R'₂ étant hydroxyalkyle ou polyhydroxyalkyle en C₁ à C₈ tels qu'ils comportent à eux deux plus de 8 OH et de préférence plus de 10 OH;
- soit la formule V ou la formule VI 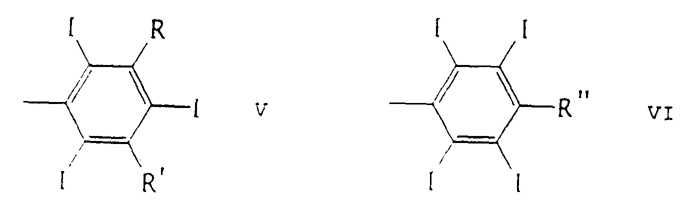 dans laquelle formule V, R et R', identiques ou différents, représentent respectivement T₂-Q₂-V₂-Ar₂ et T'₂-Q'₂-V'₂-Ar'₂ et les T₂, T'₂, Q₂, Q'₂, V₂, V'₂ ayant respectivement l'une des significations des T₁, Q₁, et V₁ et Ar₂ et Ar'₂ ayant les significations des formules III ou IV;
   et dans laquelle formule VI, R" représente T₂-Q₂-V₂-Ar₂ qui peut avoir l'une des significations données pour la formule V;
- et A est le reste biocompatible d'une molécule aliphatique ou aromatique, comportant éventuellement un ou des hétéroatomes choisis notamment parmi l'oxygène, l'azote ou les halogènes et particulièrement l'iode, le-dit reste, de masse moléculaire inférieure à 2000, ayant une structure telle que les 4 groupes Za, Zb, Zc, Zd puissent y être fixés pour former des liaisons amide ou éther-oxyde.

Par reste aliphatique, on entend un reste cyclique ou acyclique, dont les valences libres sont situées sur plusieurs atomes.

Parmi les restes A(Tz) de l'invention, on peut citer:
les dérivés tétrakis du méthane, tels que:
- C(CH₂CONH)-₄ préparé à partir du tétraacide décrit dans J. Chem. Soc. 1638 (1922)
- C(CH₂O(CH₂)₂CONH)-₄ dont l'acide correspondant est décrit dans US 2,401,607
- C(CH₂O)₄ dérivé du tétrakis(hydroxyméthyl)méthane
- C(CH₂NHCO)-₄ préparé à partir de la tétraamine décrite dans J. Chem. Soc. 1588-1595 (1938)
ou les restes tétracarboxamido, dérivés de l'acide 1,2,3,4-butyltétracarboxylique, de la triéthylènetétramine, de l'acide éthylènediaminetétraacétique et de l'acide 1,4,7,10-tétraazacyclododécane N, N', N", N'"-tétraacétique.

Par reste aromatique, on entend un reste qui comprend un ou plusieurs noyaux phényles et éventuellement des groupes alkyles en C₁ à C₄ qui peuvent être liés au noyau phényle directement ou par l'intermédiaire d'un groupe fonctionnel tel qu'un éther ou un amide, les valences libres du-dit reste pouvant être situées ou non sur les noyaux.

Parmi les restes A (Tz) qui en dérivent on peut citer:
- les tétraamido dérivés du tétrakis(carboxyphényl)méthane décrit dans Angew. Chem. Int. Ed. 25, 1097 (1986), de l'acide phényl-1,2,4,5-tétracarboxylique décrit dans J. Amer. Chem. Soc. 80, 2322 (1958) et de ses dérivés iodés, ainsi que le dérivé hexaiodé décrit dans EP-A-0 501 875, du tétrakis(aminophényl)méthane décrit dans Chem. Ber. 109, 2389 (1976) ou du tétrakis(aminométhylphényl)méthane décrit dans Angew. Chem. Int. Ed. 25, 1097 (1986).

A peut aussi comporter des hétéroatomes comme dans les amides aromatiques, par exemple les dérivés des acides oxalique, malonique et succinique de formule 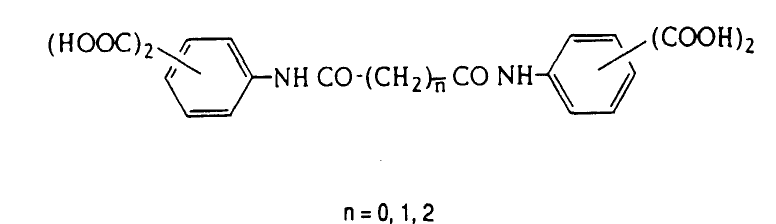 décrits dans Molecular and Biochemical Parasitology 59 201-210 (1993) qui peuvent être préparés par action de dichlorures d'acides aliphatiques sur les acides aminophényldicarboxyliques; dans le cas de leurs analogues iodés sur les noyaux phényles, il est préférable de préparer directement les tétrachlorures d'acide par action des chlorures d'acides oxalique, malonique ou succinique, par exemple, sur le dichlorure de l'acide 5-amino-2,4,6-triiodoisophtalique.

Les sels pharmaceutiquement acceptables des acides de formule I constituent également un objet de l'invention.

Les groupes alkyles peuvent être linéaires ou ramifiés.

Les groupes R'₁ et R'₂, dont dépend l'hydrophilie de la molécule, seront avantageusement hydroxylés et mieux comporteront à eux deux au moins 6 hydroxyles.

On préfère les composés dans lesquels les substituants T et V sont des groupes amides et mieux des groupes amides aromatiques secondaires; parmi ceux-ci les composés dans lesquels les substituants Q sont des alkylènes en C₁ ou C₂ sont préférés du fait de leur compacité.

Parmi les groupes A aliphatiques, on préfère les dérivés tétrakis du méthane tandis que parmi les groupes aromatiques on préfère les dérivés du tétrakiscarboxyphénylméthane, iodés ou non.

Les molécules dans lesquelles les quatre substituants de A sont identiques et mieux les deux substituants du noyau phényle triiodé lié à Z sont aussi identiques sont plus faciles d'accès.

Les composés dans lesquels Ar et Ar' sont représentés par les formules V et VI ont une rémanence vasculaire supérieure à celle de leurs homologues pour lesquels Ar et Ar' sont représentés par les formules III et IV et on choisira un type ou l'autre suivant l'indication diagnostique envisagée. Les composés de formule 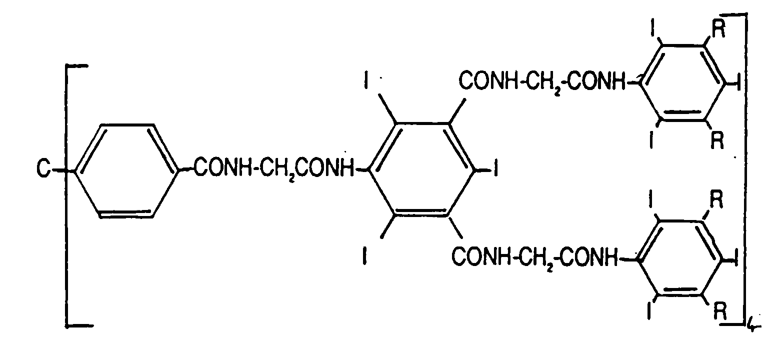 dans lesquels R représente CONR'₁R'₂, ou dans lesquels R représente 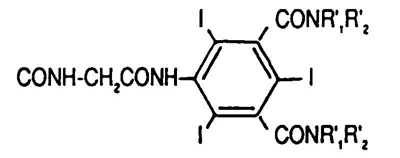 R'₁ et R'₂ étant hydroxyalkyle ou polyhydroxyalkyle en C₁ à C₈, et comportant à eux deux au moins 6 OH sont des composés particulièrement utiles comme agents de contraste pour l'observation du compartiment vasculaire du fait de leurs propriétés structurales moléculaires (volume, densité, répartition des zones hydrophobes et hydrophiles ) et de leur biocompatibilité (solubilité, toxicité) pour les doses d'iode convenant aux examens.

Le procédé de préparation des composés de formule I est un autre objet de l'invention. Ces composés peuvent être préparés en faisant réagir dès la première étape, le reste A portant les groupes fonctionnels convenables, avec le dérivé du phényle triiodé de formule 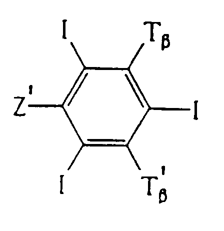 dans laquelle T_{β}, T'_{β}, Z' sont des groupes fonctionnels réactifs précurseurs des groupes T, T' et Z de la formule I, pour fixer sur le produit obtenu lors d'étapes ultérieures les substituants T₁-Q₁-V₁-Ar et T'₁-Q'₁-V'₁-Ar' simultanément lorsqu'ils sont identiques ou par étapes successives.

Néanmoins, de préférence, on prépare d'abord les composés VII portant un groupe Drécurseur de Z, de formule 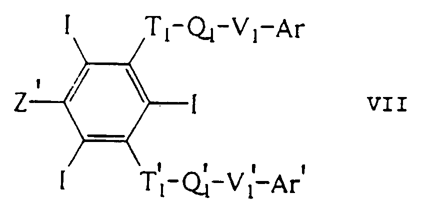 dans laquelle les T, Q, V et Ar ont la même signification que dans la formule I et Z' représente un groupe réactif précurseur de Z qui peut être COOH, COCI, NDH, OH ou Z' représente T'z-Qz-Vz, T'z étant un groupe précurseur de Tz, puis on fait réagir les composés de formule VII avec le reste A portant 4 groupes réactifs qui peuvent être selon la nature de Tz à obtenir et de Z': COOH, COCI, NDH, Cl, Br ou sulfonate ou avec A-Tz-Qz-V'z, V'z étant un groupe précurseur de Vz; les hydroxyles peuvent être éventuellement protégés au cours de ces réactions.

Dans les deux cas, la préparation met en oeuvre des techniques classiques de formation des groupes amides, à partir d'acides carboxyliques et d'amines, ou de groupes éther-oxydes à partir de phénols et d'halogénures ou de sulfonates, en tenant compte de la faible réactivité des groupes acide et chlorure d'acide carboxylique, amine et hydroxyle situés sur un noyau phényle lorsque les deux atomes de carbone adjacents portent un atome d'iode.

Plus particulièrement, le procédé selon l'invention comprend:
1) la réaction du composé de formule 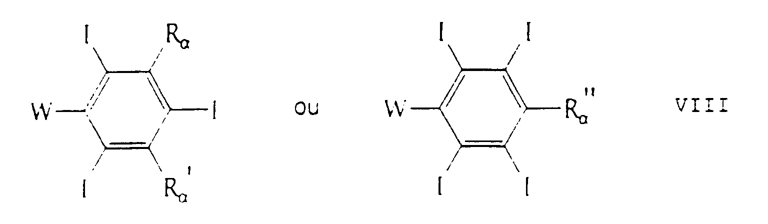 choisi selon la nature de Ar et Ar' dans le composé de formule I à obtenir, dans lesquelles R_{α}, R'_{α} et R"_{α} représentent selon le cas COCl ou NH₂ et W représente un groupe précurseur des V choisi parmi, selon le cas, ND'H, COCl et OH, avec
   - lorsque les R_{α} représentent COCl, un aminoalcool de formule HNR'₁R'₂, par exemple dans un solvant aprotique et de préférence en présence d'une base susceptible de fixer l'acide chlorhydrique libéré
   - ou lorsque les R_{α} représentent NH₂, dans des conditions classiques avec R'₂COCl ou avec R'₂COOH en présence d'un agent de couplage tel que ceux utilisés dans la chimie des peptides, les groupes hydroxyles de R'₂ étant éventuellement protégés durant la réaction d'amidification, cette réaction devant être, si nécessaire, suivie de l'alkylation de l'amide secondaire obtenue, par exemple avec R'₁Cl;
2) puis la réaction du produit obtenu avec le produit de formule Tα-Q-Vα, dans laquelle T_{α} et V_{α} sont les groupes précurseurs des T, T' et V, V' et Q représente selon le cas Q₁, Q₂, Q'₁ ou Q'₂, T_{α} étant éventuellement protégé ainsi que les groupes OH des Q, pour former les T_{α}-Q-V-Ar,
   - soit au cours d'une réaction d'amidification lorsque l'un parmi W et V_{α} est COCI, et l'autre est ND'H
   - soit lorsque W est OH et V_{α} est Cl, Br ou un sulfonate, au cours d'une réaction d'éthérification classique, en présence d'une base forte dans un solvant polaire;
3) et lorsque Ar et Ar' dans la formule I représentent la formule V ou la formule VI, la réaction d'un ou deux T_{α}-Q-V-Ar obtenu dans l'étape précédente avec, selon le cas, 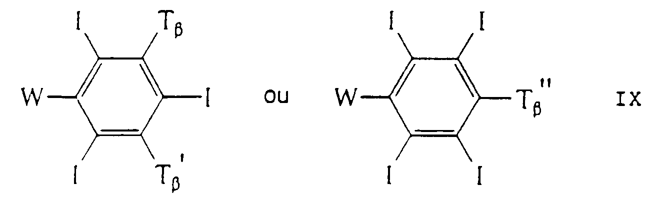 puis celle du produit obtenu avec un T_{α}-Q-V_{α} défini comme à l'étape 2, avec le produit de formule 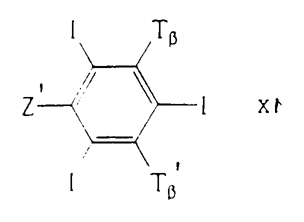 dans laquelle T_{β} et T'_{β}, identiques ou différents, sont des groupes précurseurs de T₁ et T'₁, c'est-à-dire représentent des groupes qui, par réaction avec W et W' formeront T₁ et T'₁, et Z' représente un groupe qui pourra, avec les groupes convenables situés sur A, former Za, Zb, Zc et Zd
4) ou lorsque Ar et Ar' dans la formule I représentent la formule III ou la formule IV, la réaction d'un ou deux T_{α}-Q-V_{α} -Ar avec le composé XI
5) et finalement

la réaction d'un ou plusieurs de ces groupes sur A (Z"a) (Z"b) (Z"c) (Z"d), les Z" représentant des groupes réactifs choisis pour former avec les Z' une liaison amide ou éther, et pouvant être identiques ou différents, ces réactions étant selon la nature du composé de formule I des réactions d'éthérification ou d'amidification. L'ordre des étapes peut être inversé, notamment les 1 et 2.

W peut aussi représenter T_{α}-Q-V, auquel cas l'étape 2 et selon le cas une partie de l'étape 3 sont supprimées, étant entendu que les composés VIII et IX ont été préalablement préparés par action de T_{α}-Q-V_{α} sur le dérivé du phényle tri- ou tétraiodé portant les groupes COOH, OH ou ND'H convenables.

Z' peut aussi représenter W auquel cas avant la condensation finale sur A, on pourra le modifier pour obtenir un précurseur de Z.

Lorsque R est COOH, il peut être bloqué sous forme d'ester, par exemple méthylique, dont on pourra effectuer l'hydrolyse en dernière étape avant une éventuelle salification.

Les phényles triiodés ou tétraiodés portant les fonctions acide carboxylique, amine ou phénol convenables sont connus ou peuvent être préparés par des réactions classiques. Par exemple, l'acide 5-aminotriiodoisophtalique et l'acide 3,5-diaminotriiodobenzoïque décrit dans GB 782,313 résultent de l'iodation par ICI des aminoacides correspondants; l'acide triiodotrimésique peut être préparé par diazotation, cyanation et hydrolyse de l'acide 5-aminotriiodoisophtalique tandis que l'acide 5-hydroxytriiodoisophtalique décrit dans Chem. Abs. 69 86643-6 peut être obtenu par iodation de l'acide commercial.

Les chlorures d'acide de ces composés peuvent être préparés de façon classique, par action du chlorure de thionyle, éventuellement dans un solvant neutre tel qu'un hydrocarbure chloré ou aromatique.

Les aminoalcools nécessaires pour la préparation des groupes CONR'₁R'₂ sont connus ou peuvent être préparés par des procédés d'analogie. Parmi ceux préférés dans lesquels R'₁ et R'₂ comportent chacun au moins un hydroxyle, on peut citer ceux ayant
- R'₁ = CH₂-(CHOH)₄-CH₂OH R'₂ = CH₂-(CHOH)₄-CH₂OH ou CH₂-CHOH-CH₂OH ou CH₂-CH₂OH qui sont commercialisés
- 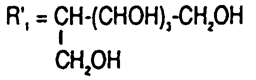 R'₂ = CH₂-CHOH-CH₂OH décrit dans EP-A-558 395
- R'₁ = R'₂ = CH₂-(CHOH)₃-CH₂OH décrit dans J. Org. Chem. 35(2) 464-7 (1970)
- R'₁ = R'₂ = CH₂-(CHOH)₂-CH₂OH décrit dans US 4,661,646

D'autres aminoalcools peuvent être préparés par disubstitution de la benzylamine avec un dérivé halogéné ou sulfoné de l'alcool convenable suivie de la débenzylation du composé obtenu, notamment par action de H₂. On peut aussi faire réagir un aldéhyde hydroxylé comme un saccharide sur un aminoalcool primaire et réduire l'imine obtenue par action de H₂, pour obtenir, par exemple, les composés avec

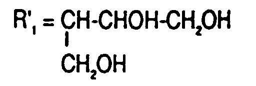

R'₂ = CH₂-CHOH-CH₂OH ou CH₂-(CHOH)₂-CH₂OH

ou

R'₁ = CH₂-(CHOH)₃-CH₂OH R'₂ = CH₂-CHOH-CH₂OH ou CH₂-(CHOH)₂-CH₂OH

Dans le cas où le noyau phényle iodé porte deux groupes chlorure d'acide ou amine, on peut préparer des composés dissymétriques en faisant réagir successivement les deux chaînes à coupler sur le noyau avec lors de la première condensation une quantité de chaîne limitée à un équivalent stoechiométrique.

Parmi les réactifs déshydratants utilisables pour la préparation des amides par réaction directe d'un acide sur l'amine convenable, on peut citer la 1-éthoxycarbonyl-2-éthoxy-1,2-dihydroquinoléine, le 1-(3-diméthylaminopropyl)3-éthylcarbodiimide ou le N,N'-dicyclohexylcarbodiimide éventuellement en présence d'hydroxybenzotriazole ou d'autres agents connus dans la chimie des peptides.

L'invention concerne aussi les produits de contraste pour la radiologie par rayons X qui comprennent comme composé absorbant les rayons au moins l'un ces composés selon l'invention.

Ces produits sous une forme pharmaceutique convenable peuvent être administrés par voie orale, rectale ou parentérale dont les voies intraveineuse, intra-artérielle, intrabronchique ou intra-arachnoïdienne; les excipients peuvent être choisis parmi ceux habituels du domaine et être associés aux additifs connus pour ajuster le pH ou l'osmolalité et diminuer certains des effets secondaires connus des dérivés iodés.

Pour une administration parentérale, notamment intravasculaire, on préfère les solutions aqueuses de pH voisin de 7, qui contiennent de 5 à 40 g d'iode fixé sur les noyaux aromatiques pour 100 ml. Suivant le type d'examen pratiqué, on pourra administrer de 5 ml à 250 ml de solution au sujet

Dans ce qui suit on décrit des exemples de composés objets de l'invention et préalablement la préparation de composés de formule VIII à XI.

A - Préparation du composé C₁ de formule VIII avec

W = T_{α}-Q-V = H₂N-CH₂-CO-NH

R_{α} = R'_{α} = CO-N(CH₂-(CHOH)₄-CH₂OH)₂

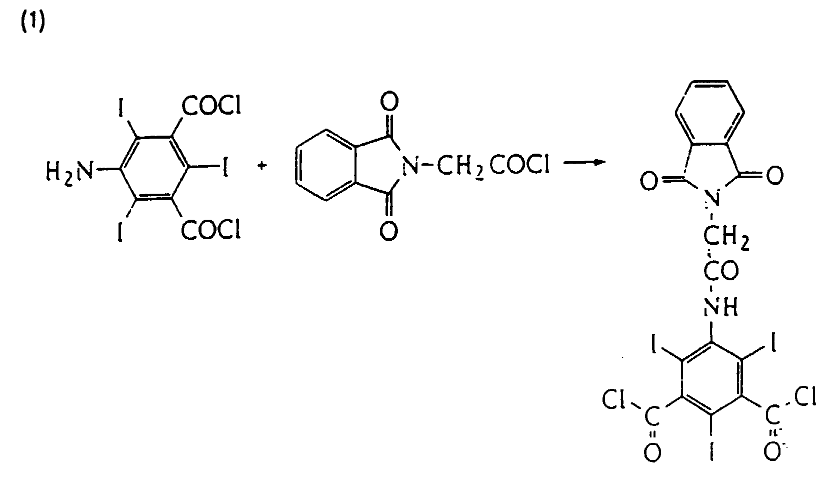

en utilisant les conditions opératoires décrites dans US 4,283,381, on obtient ce composé avec 85% de rendement. 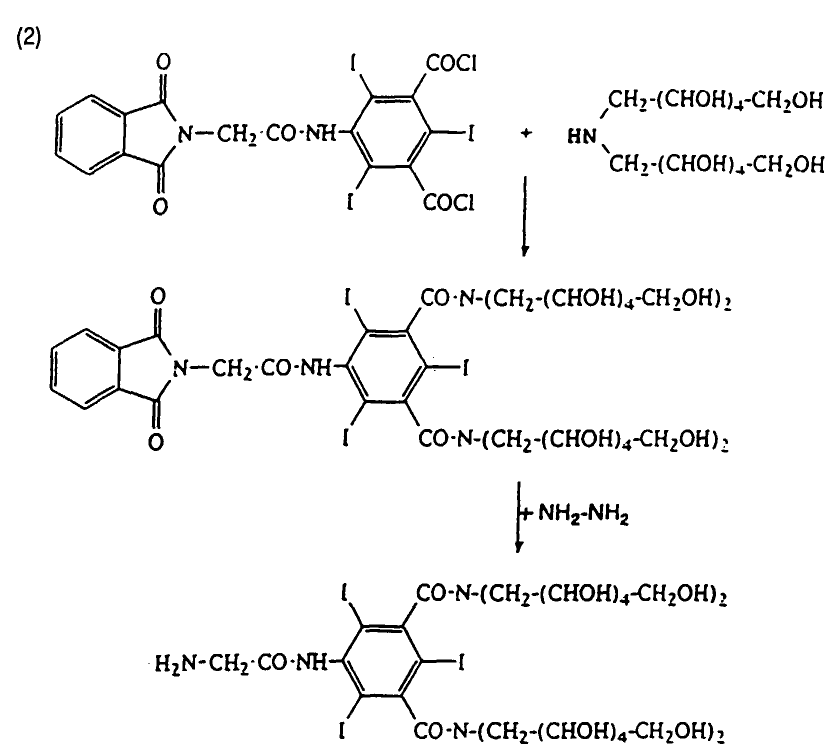 208 g du dichlorure d'acide ci-dessus, 416 g de l'amine commerciale, et 100 ml de triéthylamine sont dissous dans 2 litres de N-méthylpyrrolidone ou de diméthylacétamide et la solution est maintenue à 70° C durant 24 heures. Le précipité formé est séparé et le solvant est éliminé par distillation sous pression réduite. Le résidu dissous dans le minimum d'eau à pH 3 est passé sur une résine échangeuse d'ions cationique (1,5 l d'Amberlite® IRN77 commercialisée par Rohm et Haas sous forme acide) pour éliminer les impuretés.

A une solution du diamide ainsi obtenu dans 1,4 litre d'eau, on ajoute 33,2 ml d'hydrate d'hydrazine et on maintient le mélange à 80° C pendant 3 heures. A la température ambiante, on acidifie par addition de 53 ml d'une solution aqueuse d'acide chlorhydrique 10 N et on sépare le précipité formé.

La solution résiduelle est passée sur une colonne de résine échangeuse d'ions comprenant environ 1 litre d'Amberlite® IRA67 basique puis sur une colorne de 150 ml d'Amberlite® IRC50 sous forme acide puis greffée sur 4 litres d'Amberlite® 200C. d'où le produit cherché est élué par une solution aqueuse de NH₄OH.

L'éluat est concentré sous pression réduite. Rendement 70%.
Chromatographie HPLC: colonne LiCrosphère® C 18;5 µm (Merck) - h = 25 cm; d = 4 mm.
Eluant*: CH₃CN/P.I.C.® B8 0,05 M (Waters) 5/95; débit 1 ml/minute
Temps de rétention des isomères: 8 minutes environ
*: P.I.C. B8: mélange acide octane sulfonique/méthanol/acétate de calcium/eau.

B - Préparation du composé C₂ de formule VII avec

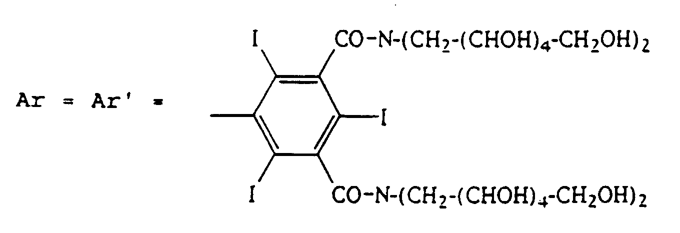

T₁-Q₁-V₁ = T'₁-Q'₁-V'₁ = CO-NH-CH₂-CONH

Z' = H₂N-CH₂-CONH

Une solution de 59 g du dichlorure d'acide décrit en A-1, 200 g de l'amine primaire VIII obtenue en A et 29,5 ml de tributylamine dans 400 ml de N-méthylpyrrolidone ou de diméthylacétamide est maintenue à 70° C pendant 24 heures. Le solvant est éliminé sous pression réduite et le résidu est chromatographié sur 3 kg de silice silanisée RP2, commercialisée par MERCK (DE) en éluant avec de l'eau ou sur 4 kg d'adsorbant XAD 1600 (commercialisé par Rohm et Haas) en éluant avec un mélange CH₃OH/H₂O.

Le produit obtenu avec 50% de rendement est traité par l'hydrate d'hydrazine, comme décrit précédemment, pour donner le composé cherché. (Rendement 45%).

Le volume d'élution de ce composé lors d'une filtration sur un gel Superdex® 30 dans une colonne de 16 mm x 60 cm, commercialisée par Pharmacia, dans un tampon à pH = 7,2 comprenant NaCl 0.1M, NaH₂PO₄ 0,05M et NaN₃ 0,01M, avec un débit de 1 ml/minute, est de 102 ml pour un échantillon injecté de 1 mg dans 250 µl de tampon.
Chromatographie HPLC: colonne Symmetry® C 18;5 µm (Waters) - h = 25 cm; d = 4,6 mm.
Eluant: CH₃CN/KH₂PO₄ aqueux 0,01 M (15/85) (sans CH₃CN pendant 5 minutes); débit 1 ml/minute
Temps de rétention des isomères: 18 minutes environ

C - Préparation du composé C₃ de formule VII avec

T₁-Q₁-V₁ = T'₁-Q'₁-V'₁ = CO-NH-CH₂-CONH

Z' = H₂N-CH₂-CONH

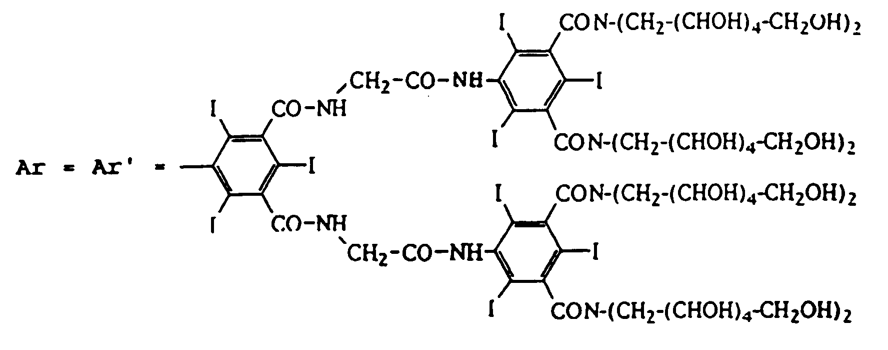

5,65 g du dichlorure d'acide de l'étape A-1, 48 g de l'amine primaire VII obtenue selon B et 3 ml de tributylamine dans 100 ml de N-méthyl pyrrolidone sont maintenus à 70° C pendant 24 heures. Après purification comme selon B, le dérivé comportant les 7 noyaux phényles iodés est traité par l'hydrate d'hydrazine pour donner l'amine primaire brute. Celle-ci est purifiée par passage sur des résines échangeuses d'ions sous forme acide et basique comme dans la préparation B suivie éventuellement d'une ultrafiltration avec une cassette Minisette de type nova commercialisée par FILTRON (USA) avec une membrane de seuil de coupure de 10 kdaltons, au cours de laquelle le produit cherché passe dans le filtrat. Rendement: 65%.

Le volume d'élution de ce produit dans les mêmes conditions opératoires que dans la préparation B est de 91 ml, alors qu'il est de 111 ml sur une colonne de Superdex® 75.
Chromatographie HPLC: colonne Symmetry® C 18;5 µm (Waters) - h = 25 cm; d = 4,6 mm.
Eluant: CH₃CN/KH₂PO₄ aqueux 0,01 M (15/85) (sans CH₃CN pendant 5 minutes); débit 1 ml/minute.
Temps de rétention des isomères: 23 minutes environ

D - Préparation du composé C₄ de formule VIII avec

W = T_{α} -Q-V = H₂N -CH₂-CONH

R_{α} = COOH

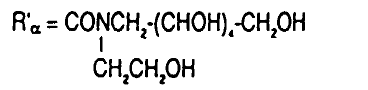

1) 239 g du dichlorure d'acide décrit dans la préparation A-1, 83 g de 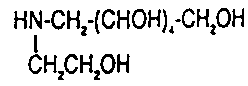 commercialisé par ALDRICH et 39 ml de triéthylamine sont dissous dans 1 l de N,N-diméthylacétamide, Après 24 heures à température ambiante, on introduit 300 ml d'eau dans le milieu qui est ensuite maintenu durant 48 heures à 45° C pour hydrolyser le chlorure d'acide résiduel. Les solvants sont alors éliminés par distillation sous pression réduite et le résidu est purifié comme précédemment par passage sur une colonne de 500 ml d'Amberlite® IRN77 et une de 3 kg de silice silanisée RP2 commercialisée par MERCK.
   On obtient ainsi le monoamide avec un rendement de 40%.
2) Ce composé est traité par rhydrate d'hydrazine et purifié par passage sur des résines échangeuses d'ions acide et basique comme dans la préparation A. Rendement 70%.

E - Préparation du composé C₅ de formule VII avec

T₁-Q₁-V₁ = T'₁-Q'₁-V'₁ = CO-NH-CH₂-CONH

Z' = H₂N-CH₂-CONH

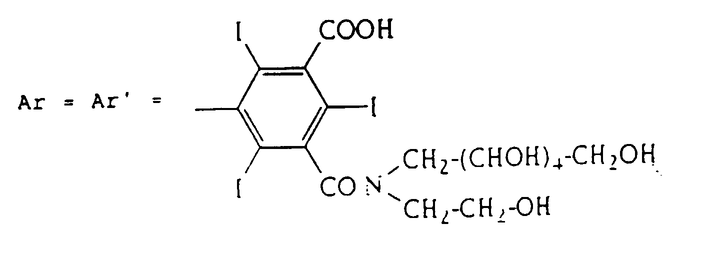

Avec des conditions opératoires analogues à celles décrites pour la préparation B, on obtient le composé recherché avec 30% de rendement.

Son volume d'élution dans les conditions précédemment indiquées sur Superdex® 30 est de 92 ml.

### Exemple 1

Composé No. 1 de formule I dans laquelle

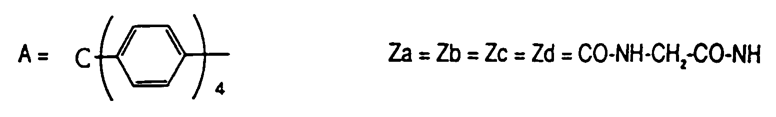

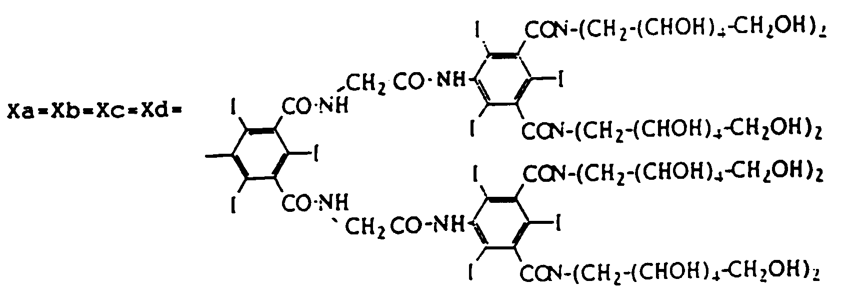

Une solution de 15,3 g du composé obtenu selon la préparation B, 0,486 g de tétrakis(carboxyphényl)méthane, 0,768 g de 1-hydroxybenzotriazole, 1,09 g de 1-(3-diméthylaminopropyl)3-éthylcarbodiimide et 1 ml de triéthylamine dans 100 ml de diméthylformamide est portée à 40° C et cette température est maintenue durant 6 heures avant élimination du solvant par distillation sous pression réduite.

Le résidu est dissous dans le minimum d'eau et soumis à une dia-ultrafiltration avec une cassette Minisette® de type nova commercialisée par FILTRON (USA) avec une membrane en polyéther sulfone de seuil de coupure de 5 kdaltons. Rendement 95%.

Le volume d'élution pour ce composé, déterminé dans les mêmes conditions de filtration sur gel que précédemment est de 49 ml (Superdex® 30).

### Exemple 2

Composé No. 2 de formule I dans laquelle

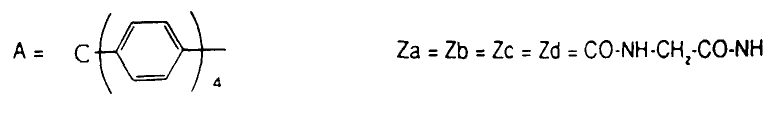

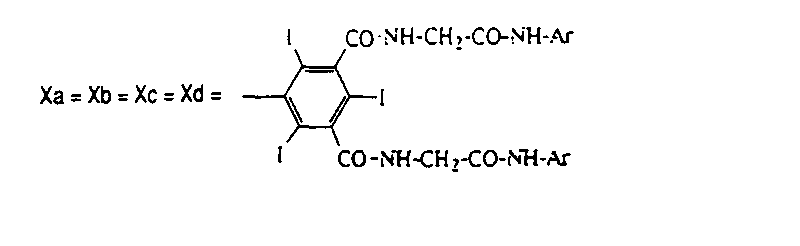

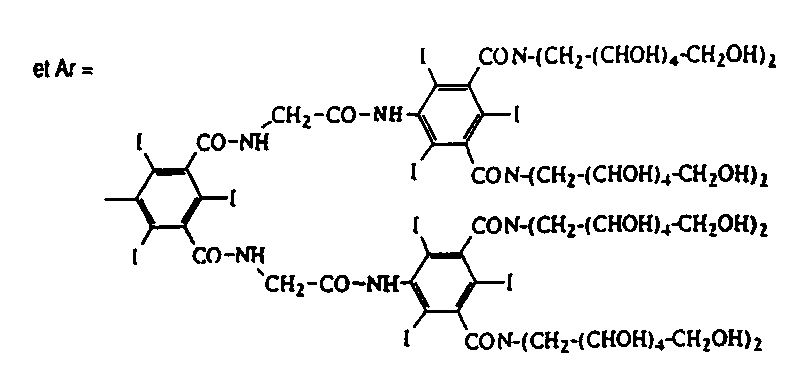

Le composé préparé en C est mis à réagir avec le tétrakis(carboxyphényl)méthane dans des conditions opératoires équivalentes à celles décrites dans rexemple précedent, à l'exception de l'ultrafiltration qui a lieu sur une membrane de seuil de coupure 10 kdaltons.

Le composé de formule I, obtenu avec 90% de rendement, a un volume d'élution de 85 ml lors d'une filtration sur gel dans les conditions décrites précédemment (Superdex® 75).

### Exemple 3

Composé No. 3 de formule I dans laquelle

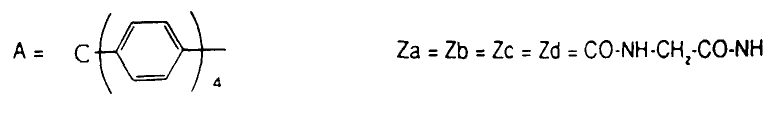

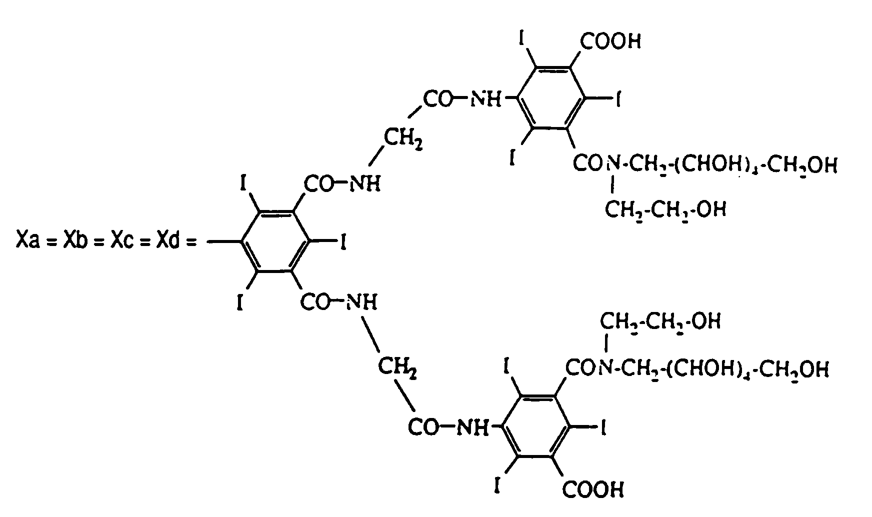

20 g du composé VII obtenu dans la préparation E, 0,89 g du tétrakis(carboxyphényl)méthane, 1,41 g de 1-hydroxybenzotriazole et 2 g de 1-(3-diméthylaminopropyl)3-éthylcarbodiimide et 1,5 ml de triéthylamine sont dissous dans 200 ml de diméthylformamide et le milieu réactionnel est maintenu à 40° C durant 6 heures. Après élimination du solvant, le résidu est dissous dans l'eau puis soumis à une ultrafiltration comme précédemment mais avec une membrane de seuil de coupure de 3 kdaltons.

Le produit final précipite par addition de HCI concentré au milieu.

Rendement 87%.

Son volume d'élution, déterminé dans les conditions de filtration sur gel précédentes est de 49 ml. (Superdex® 30).

### Exemple 4

Préparation des composés No. 1 et 2 dans le diméthylacétamide.

On dissout lentement dans le diméthylacétamide à une température comprise entre 20 et 60° C environ, 0,012 mole du composé C₂ ou du composé C₃, à raison de 25 g/100 ml. On introduit ensuite à température ambiante 0.0025 mole de tétrakis-(carboxyphényl)méthane, 0.014 mole de N-N'-dicyclohexylcarbodiimide, 0.014 mole de 1-hydroxybenzotriazole et 0,015 mole de triéthylamine. Après 24 heures d'agitation, on ajoute 5 volumes d'eau et la solution est ultrafiltrée comme précédemment pour donner avec 55% de rendement le produit cherché sous forme d'une poudre blanche.

Ces composés peuvent être caractérisés par chromatographie d'exclusion stérique (CES) sur 4 colonnes montées en série commercialisées par SHODEX (JP) sous les références OHpaK SB-8.. HQ de diamètre 8 mm et de longueur 30 cm, contenant un gel de polyhydroxyméthacrylate: SB-804 (limite d'exclusion = 10⁶ daltons, standard pullulan) + SB-803 (10⁵) + SB-802-5 (10⁴) + SB802-5. L'éluant est un mélange de solution aqueuse de NaCI 0,16 M et acétonitrile (70/30 V/V); débit 0,8 ml/minute; température 30° C.

Le temps de rétention t_{R} du composé No. 1 est de 34,3 minutes alors que celui du composé C₂ de départ est de 38,7 minutes.

Pour le composé No. 2, t_{R} = 31,8 minutes et pour C₃, t_{R} = 36,5 minutes.

## Revendications

1. Composés de formule 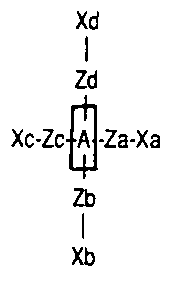 dans laquelle
Za, Zb, Zc, Zd, identiques ou différents, représentent Tz-Qz-Vz et Tz et Vz, identiques ou différents, sont choisis parmi les groupes O,CO-ND ou ND-CO avec D représente H, alkyle, hydroxyalkyle ou polyhydroxyalkyle en C₁ à C₆ et Q représente alkylène, hydroxyalkylène ou polyhydroxyalkylène en C₁ à C₆ ou Qz et/ou Tz n'existent pas,
- Xa, Xb, Xc, Xd, identiques ou différents, représentent 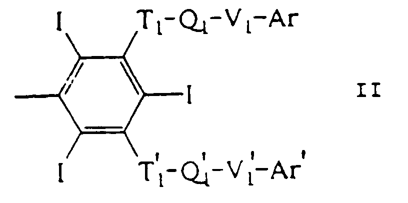 et
· T₁, T'₁, V₁, V'₁, identiques ou différents, représentent CO-ND' ou ND'-CO avec D' représentant H ou alkyle en C₁ à C₆ portant, éventuellement, un ou plusieurs groupes OH;
· Q₁, et Q'₁, identiques ou différents, représentent alkylène en C₁ à C₆ portant, éventuellement, un ou des OH;
· Ar et Ar', identiques ou différents, représentent soit la formule III ou la formule IV: 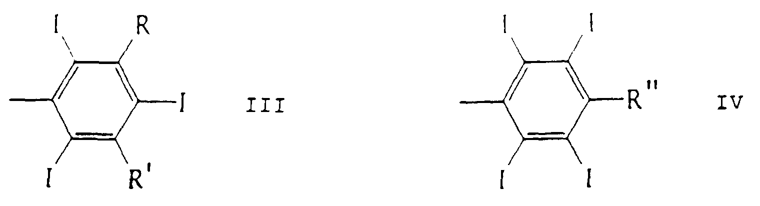 dans laquelle formule III R est COOH et R' est CO-NR'₁ R'₂, ou N(R'₁)-CO- R'₂, R'₁ et R'₂ étant choisis parmi H, alkyle, hydroxyalkyle ou polyhydroxy-alkyle en C₁ à C₈, de telle sorte qu'ils comportent à eux deux plus de 4 OH ou dans laquelle R et R', identiques ou différents, représentent CO-NR'₁R'₂ ou N(R'₁)-CO-R'₂ et R'₁ et R'₂ sont H, alkyle, hydroxyalkyle ou polyhydroxyalkyle en C₁ à C₈ et R et R' comportent à eux deux plus de 6 groupes OH,
et dans laquelle formule IV, R" représente CO-NR'₁R'₂ ou N(R'₁)-CO-R'₂, R'₁ et R'₂ étant hydroxyalkyle ou polyhydroxyalkyle en C₁ à C₈, tels qu'ils comportent à eux deux plus de 8 OH, soit la formule V ou la formule VI 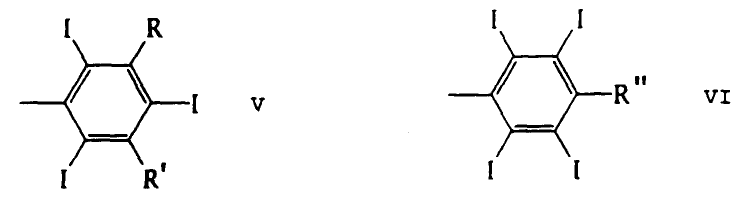 dans laquelle R et R', identiques ou différents représentent respectivement T₂-Q₂-V₂-Ar₂ et T'₂-Q'₂-V'₂-Ar'₂ et les T₂, T'₂, Q₂, Q'₂, V₂, V'₂ ayant respectivement l'une des significations des T₁, Q₁, et V₁et Ar₂ et Ar'₂ ayant les significations des formules III ou IV,
et dans laquelle formule VI, R" représente T₂-Q₂-V₂-Ar₂ qui peut avoir l'une des significations données pour la formule V
- et A est le reste biocompatible d'une molécule aliphatique ou aromatique, de masse moléculaire inférieure à 2000, dont les 4 valences libres peuvent former un groupe amide ou éther avec Za, Zb, Zc ou Zd,
ainsi que leurs sels avec des bases pharmaceutiquement acceptables.

2. Composés selon la revendication 1, caractérisés en ce que les 4 substituants de A sont identiques.

3. Composés selon l'une des revendications 1 et 2 caractérisés en ce que R'₁ et R'₂ sont hydroxylés et comprennent à eux deux plus de 8 OH.

4. Composés selon l'une des revendications 1 et 2 caractérisés en ce que R'₁ et R'₂ sont hydroxylés et comprennent à eux deux 10 OH.

5. Composés selon l'une des revendications l à 4, caractérisés en ce que les T et V sont des groupes CO-NH ou NH-CO.

6. Composés selon l'une des revendications précédentes, caractérisés en ce que les T et V sont des groupes CO-NH ou NH-CO et les Q des alkylènes en C₁-C₂.

7. Composés selon l'une des revendications précédentes, caractérisés en ce que A est un radical aromatique dérivé du tétrakisphénylméthane.

8. Composés selon l'une des revendications 1 à 6, caractérisés en ce que A est le reste du tétrakis(carboxyphényl)méthane.

9. Composés selon l'une des revendications 2 à 5, caractérisés en ce que A(Tz)₄ est un radical de formule 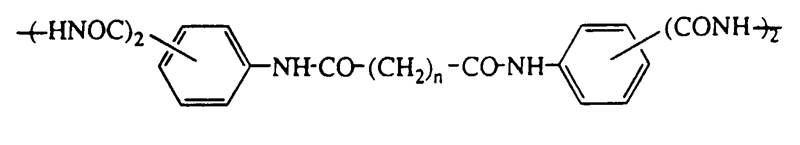 dans laquelle n = 0, 1, 2 et dont les phényles sont éventuellement iodés.

10. Composés de formule I selon l'une des revendications précédentes, caractérisés en ce que Ar et Ar' représentent la formule III

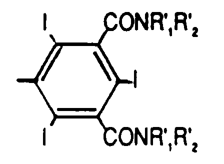

11. Composés de formule I selon l'une des revendications précédentes, caractérisés en ce que Ar et Ar' représentent 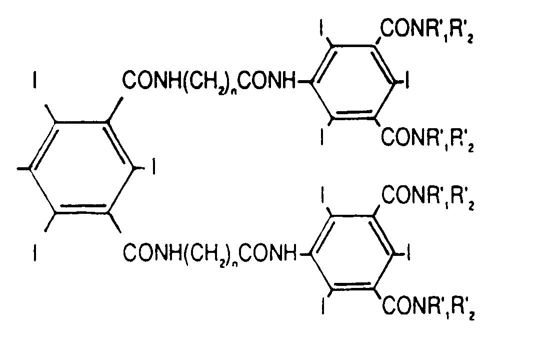 avec n = 1 ou 2.

12. Procédé de préparation des composés de formule I qui consiste à faire réagir sur le reste A portant 4 groupes réactifs, qui sont selon le cas COOH, COCI, NDH, halogène ou sulfonate,
- soit avec le composé de formule 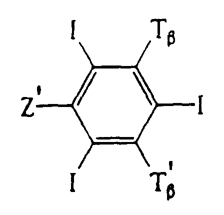 dans laquelle T_{β}, T'_{β}, Z' sont des groupes fonctionnels réactifs précurseurs des groupes T₁, T'₁, et Z de la formule I, auquel cas on fixe ensuite sur le produit obtenu les substituants T₁-Q₁- V₁-Ar et T'₁-Q'₁-V'₁-Ar' directement ou par étapes successives,
- soit avec le composé de formule 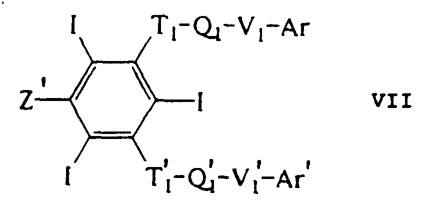 dans laquelle les T, Q, V et Ar ont la même signification que dans la formule I définie à la revendication 1 et Z' représente un groupe précurseur de Z qui peut être COOH, COCI, NDH, OH ou T'z-Qz- Vz, T'z étant un groupe précurseur de Tz.

13. Compositions de produit de contraste pour la radiologie par rayons X qui comportent une quantité efficace d'un composé selon l'une des revendications 1 à 11, et un excipient pharmaceutiquement acceptable.

## Patentansprüche

1. Verbindungen der Formel 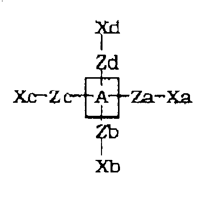 in der
- Za, Zb, Zc, Zd, die gleichartig oder verschieden sind, Tz-Qz-Vz darstellen, worin Tz und Vz. die gleichartig oder verschieden sind, ausgewählt sind aus den Gruppen O, CO-ND oder ND-CO, worin D H, C₁-C₆-Alkyl, C₁-C₆-Hydroxyalkyl odcr C₁-C₆-Polyhydroxyalkyl und Q C₁-C₆-Alkylen, C₁-C₆-Hydroxyalkylen oder C₁-C₆-Polyhydroxyalkylen darstellen, oder Qz und/oder Tz nicht vorhanden sind,
- Xa, Xb, Xc, Xd, die gleichartig oder verschieden sind, 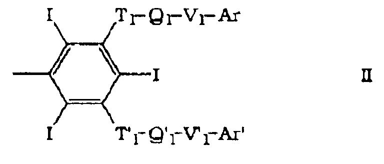 darstellen,
worin
· T₁, T'₁, V₁, V'₁, die gleichartig oder verschieden sind, CO-ND' oder ND'-CO bedeuten, worin D' H oder C₁-C₆-Alkyl, welches gegebenenfalls eine oder mehrere OH-Gruppen trägt, bedeutet;
· Q₁ und Q'₁, die gleichartig oder verschieden sind, C₁-C₆-Alkylen, welches gegebenenfalls eine oder mehrere OH-Gruppen trägt, darstellen;
· Ar und Ar', die gleichartig oder verschieden sind, entweder für die Formel III oder dic Formel IV stehen: 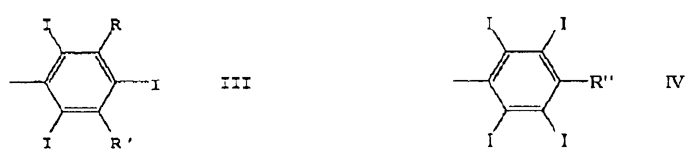 in welcher Formel III R COOH und R' CO-NR'₁R'₂ oder N(R'₁)-CO-R'₂ darstellen, worin R'₁ und R'₂ aus H, C₁-C₈-Alkyl, C₁-C₈-Hydroxyalkyl und C₁-C₈-Polyhydroxyalkyl ausgewählt sind, derart, daß diese beiden Gruppen zusammen mehr als 4 OH-Gruppen aufweisen, oder worin R und R', die gleichartig oder verschieden sind, CO-NR'₁R'₂ oder N(R'₁)-CO-R'₂ darstellen, worin R'₁ und R'₂ H, C₁-C₈-Alkyl, C₁-C₈-Hydroxyalkyl oder C₁-C₁-Polyhydroxyalkyl bedeuten und die beiden Gruppen R und R' zusammen mehr als 6 OH-Gruppen aufweisen,
und in der Formel IV R" CO-NR'₁R'₂ oder N(R'₁)-CO-R'₂ darstellt, worin R'₁ und R'₂ C₁-C₈-Hydroxyalkyl oder C₁-C₈-Polyhydroxyalkyl bedeuten, derart, daß diese beiden Gruppen gemeinsam mehr als 8 OH-Gruppen aufweisen,
oder die Formel V oder die Formel VI bedeuten 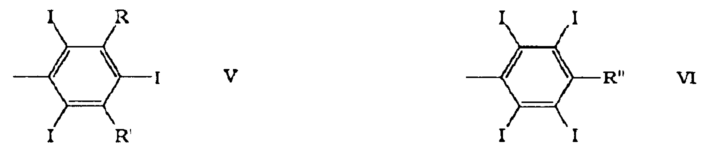 worin R und R', die gleichartig oder verschieden sind, T₂-Q₂-V₂-Ar₂ bzw. T'₂-Q'₂-V'₂-Ar'₂ bedeuten, wobei T₂, T'₂, Q₂, Q'₂, V₂ und V'₂ jeweils eine der Bedeutungen von T₁, Q₁ bzw. V₁ besitzen und Ar₂ und Ar'₂ die Bedeutungen der Formeln III oder IV besitzen,
und in welcher Formel VI R" T₂-Q₂-V₂-Ar₂ darstellt, welche eine der für die Formel V angegebenen Bedeutungen besitzen kann,
- und A den biologisch verträglichen Rest eines aliphatischen oder aromatischen Moleküls darstellt mit einer Molekülmasse von weniger als 2000, dessen 4 freie Valenzen mit Za, Zb, Zc oder Zd eine Amidgruppe oder eine Ethergruppe bilden können,
sowie deren Salze mit pharmazeutisch annehmbaren Basen.

2. Verbindungen nach Anspruch 1, dadurch gekennzeichnet, daß die 4 Substituenten von A identisch sind.

3. Verbindungen nach einem der Ansprüche 1 und 2, dadurch gekennzeichnet, daß R'₁ und R'₂ hydroxyliert sind und diese beiden Gruppen gemeinsam mehr als 8 OH-Gruppen aufweisen.

4. Verbindungen nach einem der Ansprüche 1 und 2, dadurch gekennzeichnet, daß R'₁ und R'₂ hydroxyliert sind und diese beiden Gruppen gemeinsam 10 OH-Gruppen aufweisen.

5. Verbindungen nach einem der Ansprüche 1 bis 4, dadurch gekennzeichnet, daß T und V Gruppen der Formeln darstellen: CO-NH oder NH-CO.

6. Verbindungen nach einem der vorhergehenden Ansprüche, dadurch gekennzeichnet, daß T und V Gruppen der Formeln CO-NH oder NH-CO darstellen und Q C₁-C₂-Alkylengruppen bedeuten.

7. Verbindungen nach einem der vorhergehenden Ansprüche, dadurch gekennzeichnet, daß A ein von Tetrakisphenylmethan abgeleiteter aromatischer Rest ist.

8. Verbindungen nach einem der Ansprüche 1 bis 6, dadurch gekennzeichnct, daß A für den Tetrakis(carboxyphenyl)-methanrest steht.

9. Verbindungen nach einem der Ansprüche 2 bis 5, dadurch gekennzeichnet, daß A(Tz)₄ eine Gruppe der Formel ist 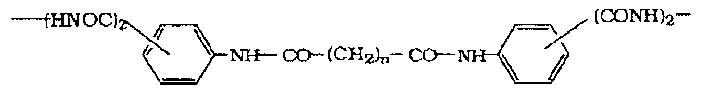 in der n 0, 1 oder 2 bedeutet und worin die Phenylreste gegebenenfalls iodiert sind.

10. Verbindungen der Formel I nach einem der vorhergehenden Ansprüche, dadurch gekennzeichnet, daß Ar und Ar' der Formel III entsprechen:

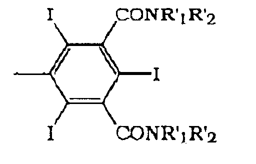

11. Verbindungen der Formel I nach einem der vorhergehenden Ansprüche, dadurch gekennzeichnet, daß Ar und Ar' der Formel 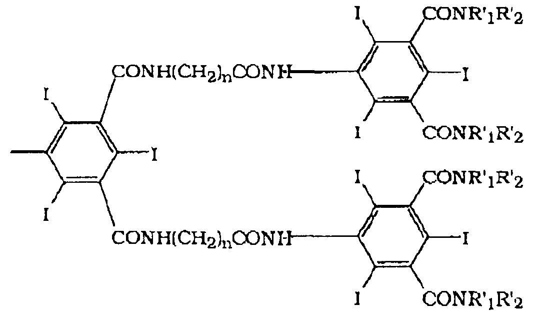 entsprechen, worin n 1 oder 2 darstellt.

12. Verfahren zur Herstellung der Verbindungen der Formel 1, welches darin besteht, den Rest A, der 4 reaktive Gruppen aufweist, die je nachdem Gruppen der Formeln COOH, COCl, NDH, Halogenatome oder Sulfonatgruppen sind,
- entweder mit der Verbindung der Formel 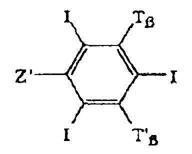 in der T_{β}, T'_{β} und Z' reaktive funktionelle Gruppen sind. die Vorläufer sind der Gruppen T₁, T'₁ und Z der Formel I, in welchem Fall anschließend die Substituenten T₁-Q₁-V₁-Ar und T'₁-Q'₁-V'₁-Ar' direkt oder in aufeinanderfolgenden Stufen an das erhaltene Produkt gebunden werden,
- oder mit der Verbindung der Formel 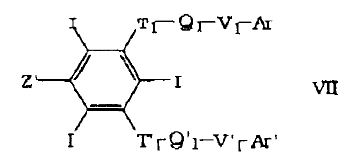 in der die Gruppen T, Q, V und Ar die bezüglich der Formel I in Anspruch 1 angegebenen Bedeutungen besitzen und Z' eine Vorläufergruppe für Z darstellt, welche eine Gruppe der Formel COOH, COCl, NDH, OH oder T'z-Qz-Vz sein kann, worin T'z eine Verläufergruppe von Tz darstellt, umzusetzen.

13. Kontrastmittelzusammensetzungen für die Röntgenstrahlen-Radiologie enthaltend eine wirksame Menge einer Verbindung nach einem der Ansprüche 1 bis 11 und ein pharmazeutisch annehmbares Trägermaterial.

## Claims

1. Compounds of the formula 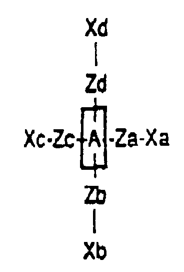 wherein
Za, Zb, Zc, Zd, either identical or different, represent Tz-Qz-Vz and Tz and Vz, either identical or different, are selected from groups O,CO-ND or ND-CO where D represents H, alkyl, hydroxy alkyl or polyhydroxy alkyl in C₁ to C₄, and
Q represents alkylene, hydroxy alkylene or polyhydroxy alkylene in C₁ to C₄, or Qz and/or Tz do not exist,
Xa, Xb, Xc, Xd, either identical or different, represent 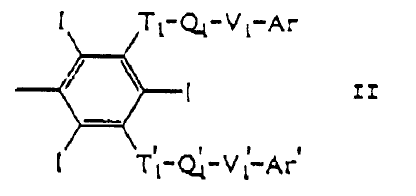 and
T₁, T'₁, V₁, V'₁, either identical or different, represent CO-ND' or ND'-CO where D' represents H or alkyl in C₁ to C₄, possibly having one or several OH groups,
Q₁ and Q'₁, either identical or different, represent alkylene in C₁ to C₄, possibly having one or some OH groups,
Ar and Ar', either identical or different, represent either formula III or formula IV: 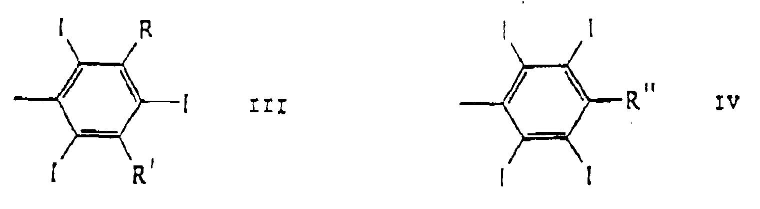
in which formula III R is COOH and R' is CO-NR'₁, R'₂ or N(R'₁)-CO-R'₂, R'₁ and R'₂ being selected from H, alkyl, hydroxy alkyl or polyhydroxy alkyl in C₁ to C₄, such that they both comprise more than 4 OH, and in which R and R', either identical or different, represent CO-NR'₁, R'₂ or N(R'₁)-CO-R'₂, and R'₁ and R'₂ are H, alkyl, hydroxy alkyl or polyhydroxy alkyl in C₁ to C₄, and R and R' both comprise more than 6 OH groups,
and in which formula IV R" represents CO-NR'₁, R'₂ or N(R'₁)-CO-R'₂, R'₁ and R'₂ being hydroxy alkyl or polyhydroxy alkyl in C₁ to C₄, such that they both comprise more than 8 OH, either formula V or formula VI 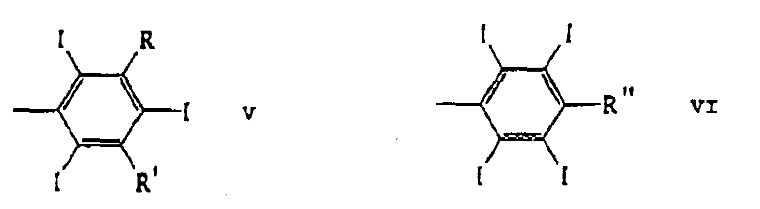
in which R and R', either identical or different, respectively represent T₂-Q₂-V₂-Ar₂ and T'₂-Q'₂-V'₂-Ar'₂, and T₂-T'₂-Q₂-Q'₂-V₂-V'₂ respectively have one of the meanings of T₁, Q₁ and V₁ and Ar₂ or Ar'₂ having the meanings of formulas III and IV,
and in which formula VI R" represents T₂-Q₂-V₂-Ar₂ with one of the meanings given for formula V
and A is the biocompatible residue of an aliphatic or aromatic molecule with a molecular mass of less than 2000,
of which the 4 free valencies may form an amide or ether group with Za, Zb, Zc or zd,
as well as their salts with pharmaceutically acceptable bases.

2. Compounds according to Claim 1, characterised in that the 4 substituents of A are identical.

3. Compounds according to one of Claims 1 and 2, characterised in that R'₁ and R'₂ are hydroxylated and both comprise more than 8 OH.

4. Compounds according to one of Claims 1 and 2, characterised in that R'₁ and R'₂ are hydroxylated and both comprise 10 OH.

5. Compounds according to one of Claims 1 to 4, characterised in that the T and V values are CO-NH or NH-CO groups.

6. Compounds according to one of the preceding claims, characterised in that the T and V values are CO-NH or NH-CO groups and the Q values are alkylenes in C₁-C₂.

7. Compounds according to one of the preceding claims, characterised in that A is an aromatic radical derived from tetrakisphenyl methane.

8. Compounds according to one of Claims 1 to 6, characterised in that A is the residue of the tetrakis(carboxyphenyl) methane.

9. Compounds according to one of Claims 2 to 5, characterised in that A(Tz)₄ is a radical of the formula 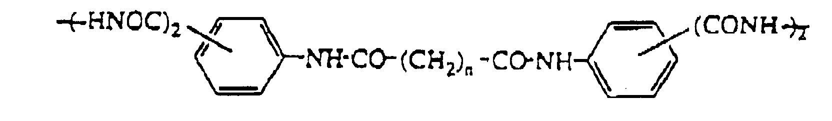 in which n = 0, 1, 2 and the phenyls of which are possibly iodised.

10. Compounds of formula I according to one of the preceding claims, characterised in that Ar and Ar' represent formula III

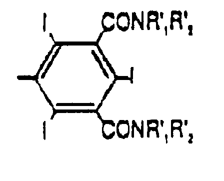

11. Compounds of formula I according to one of the preceding claims, characterised in that Ar and Ar' represent 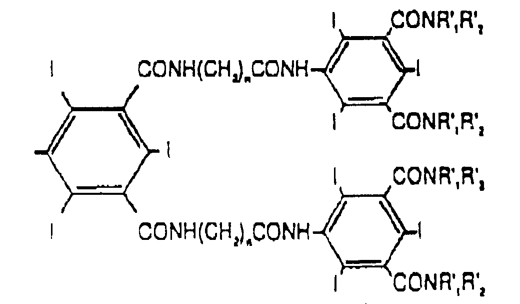 where n = 1 or 2.

12. Process for the preparation of compounds of formula I which consists in causing a reaction on the A residue having 4 reactive groups, which are COOH, COCl, NDH, halogen or sulphonate according to the respective case, either with the compound of formula 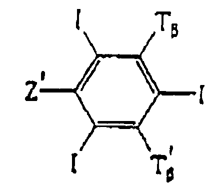 in which T_{β}, T'_{β}, Z' are the precursor reactive functional groups of groups T₁, T'₁, and Z of formula I, in which case attention is then focused on the product obtained from substituents T₁-Q₁-V₁-Ar and T'₁-Q'₁-V'₁-Ar' either directly or in successive stages,
or with the compound of formula 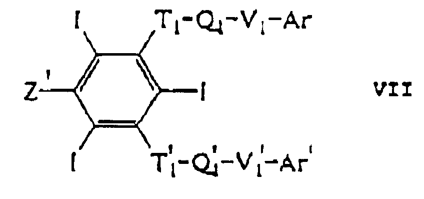 in which T, Q, V and Ar have the same meaning as in formula I defined in Claim 1, and Z' represents a precursor group of Z which may be COOH, COCl, NDH, OH or T'z-Qz-Vz, T'z being a precursor group of Tz.

13. Compositions for a contrast medium for X ray radiography which contain an effective quantity of a compound according to one of Claims 1 to 11 and a pharmaceutically acceptable excipient.
